# FASCICULE DE BREVET EUROPEEN

(11) **EP 1 166 718 B1**
(45) Date de publication et mention de la délivrance du brevet: **01.12.2004**
(21) Numéro de dépôt: 01390006.3
(22) Date de dépôt: 19.06.2001
(51) Int. Cl.: A61B 5/103

(54) **Dispositif d'analyse des troubles de l'équilibre et de la posture d'un individu**
Vorrichtung zur Analyse von Gleichgewichtsstörungen und der Körperhaltung eines Subjektes
Device for the analysis of balance disturbancies and of the posture of a subject

(30) Priorité: 23.06.2000 FR 0008064
(43) Date de publication de la demande: 02.01.2002
(73) Titulaire: Patrick Savet Sarl, 31700 Blagnac (FR)
(72) Inventeur: Savet, Patrick, 31700 Blagnac (FR)
(74) Mandataire: Cabinet BARRE LAFORGUE & associés

(56) Documents cités:
- US-A- 4 195 643
- US-A- 5 627 327

## Description

L'invention concerne un dispositif d'analyse des troubles de l'équilibre et des stratégies de régulation posturale d'un individu.

Il est universellement reconnu à l'heure actuelle par le corps médical que l'analyse des troubles de l'équilibre est un excellent outil pour notamment apprécier et objectiver les dysfonctionnements des systèmes sensoriels et neuro-moteurs principaux, et également pour évaluer l'efficacité d'une thérapie rééducative de ces mêmes systèmes.

Pour ces raisons, les scientifiques et médecins ont développé des dispositifs et des méthodologies pour réaliser cette analyse. L'ensemble de ces techniques est connu sous le nom de posturologie clinique, et vise à permettre l'analyse de façon rigoureuse et fiable, de la posture d'un individu dans des positions statiques et dynamiques.

A cet effet, les équipements développés consistent à l'heure actuelle en des plates-formes informatisées de posturologie qui permettent de mesurer et d'enregistrer le déplacement du centre des pressions podales d'un individu dans différentes conditions d'examen, déplacement qui est une conséquence directe des oscillations du corps, elles-mêmes résultantes de la qualité de régulation des différents systèmes neuro-sensoriels et neuro-moteurs.

Les plates-formes les plus couramment utilisées ont pour but de permettre d'apprécier quantitativement les mouvements spontanés du corps d'un individu en position orthostatique sur une base stable.

A cet effet, les plates-formes consistent généralement en des plates-formes de forces statiques comportant un plateau indéformable reposant sur des capteurs rigides (jauges de contrainte) permettant d'analyser la composante verticale de la force résultante appliquée par le poids du corps d'un individu sur lesdites plates-formes.

De telles plates-formes reliées à un micro-ordinateur permettent notamment de fournir plusieurs paramètres (X moyen, Y moyen, longueur, surface, transformées rapides de FOURIER) qui rendent compte des performances d'un individu à tenir debout à un moment donné, et permettent d'apprécier la qualité de ses boucles de régulation sensori-motrices.

Les paramètres recueillis grâce à de telles plates-formes statiques se sont toutefois avérés insuffisants du fait que, dans la vie courante, un sujet est rarement en situation d'équilibre statique. A cet effet, et en vue de compléter les résultats obtenus au moyen des plates-formes statiques, il a été développé des plates-formes instables autorisant des déplacements du polygone de sustentation sous l'effet des mouvements physiologiques d'équilibration propres du sujet, donc indépendants de toute motorisation externe.

Un premier type de plate-forme mobile utilisée actuellement consiste en un plateau reposant sur une rotule ou sur un axe (plates-formes de SINGER, BACHMAN, BEGBIE ...). Toutefois, il s'est avéré que lorsqu'un sujet est en place sur de telles plates-formes, il se trouve en équilibre trop instable, et que, de ce fait, il risque de chuter. Le maintien de l'équilibre sur ce type de plateau est, en outre, impossible pour des sujets pathologiques.

Une plate-forme mobile disposée sur une plate-forme statique est décrite en US 5 627 327 A.

Dans la pratique, et pour ces raisons, de telles plates-formes mobiles sont équipées soit de butées de limitation de l'inclinaison du plateau, soit d'un frein, d'un amortisseur ou d'un ressort de ralentissement du mouvement, dans le but d'éviter la chute du sujet.

Toutefois, le jeu de butées fragmente la continuité de la mesure, et le recours à des freins, amortisseurs ou ressorts compromet la reproductibilité de l'examen, de sorte que les résultats obtenus au moyen de telles plates-formes sont difficiles à exploiter et ne représentent en aucun cas une preuve d'équilibration dynamique physiologique.

Pour pallier ces inconvénients, et en vue d'obtenir des paramètres de mesure de l'équilibration dynamique faciles à comparer, une solution a consisté à réaliser une plate-forme mobile créant une situation d'équilibre spontanément instable, constituée d'un plateau sous lequel est fixée une portion de cylindre.

Selon ce principe, l'ensemble repose sur le sol par l'intermédiaire d'une génératrice du cylindre et forme une plate-forme à un seul degré de liberté de mouvement. La variation d'inclinaison imposée par le sujet afin de se maintenir en équilibre est captée à l'aide d'un inclinomètre ou d'un accéléromètre qui donne directement la position angulaire de la plate-forme par rapport à l'horizontale, donnée qui est acquise et traitée par un micro-ordinateur.

Une telle plate-forme mobile permet ainsi le développement par le sujet de réactions d'équilibration simples et efficaces composées d'une rotation associée à une translation du sujet sur la plate-forme.

Grâce à sa simplicité, à sa facilité de mise en oeuvre, à la possibilité de découpler équilibre frontal et équilibre sagittal, et aux nombreux paramètres exploitables, une telle plate-forme mobile s'est avérée constituer, en complément des plates-formes statiques, un excellent moyen d'évaluation des troubles de l'équilibre, de suivi objectif des résultats d'une thérapie médicale ou chirurgicale et de guidage d'une physiothérapie.

La présente invention vise également un dispositif comportant une plate-forme mobile telle que ci-dessus décrite, et a pour principal objectif de fournir un dispositif doté d'une telle plate-forme, d'une part, permettant d'effectuer l'analyse de la posture d'un individu tant dans des conditions orthostatiques que dans des conditions dynamiques, et d'autre part, dont le prix de revient n'est que légèrement supérieur à celui d'une plate-forme statique seule.

Un autre objectif de l'invention est de fournir un dispositif d'analyse permettant de recueillir un nombre de paramètres accru, dans les conditions dynamiques, par rapport à ceux recueillis avec les plates-formes mobiles actuelles, et différenciant les stratégies d'équilibration sur le plan sagittal avec les stratégies d'équilibration sur le plan frontal.

A cet effet, l'invention vise un dispositif d'analyse de la posture d'un individu, comprenant :
- une plate-forme de forces statique dotée de capteurs aptes à délivrer des signaux représentatifs de la position et de la valeur absolue des forces exercées sur ladite plate-forme par un individu en position d'équilibre orthostatique,
- une plate-forme mobile disposée sur la plate-forme statique et constituée d'un plateau fixé sur des moyens supports présentant une face inférieure d'appui en forme de secteur cylindrique dont la zone de contact avec la plate-forme statique consiste en une des génératrices de ladite face inférieure d'appui formant le pivot de ladite plate-forme mobile,
- une unité d'acquisition et de traitement des signaux délivrés par les capteurs de la plate-forme statique, programmée pour acquérir lesdits signaux avec une fréquence prédéterminée et pour procéder à une analyse des troubles de l'équilibre et de la posture de l'individu selon des plans d'équilibration frontal et sagittal, et à des analyses fréquentielles des oscillations engendrées de façon à définir la contribution des différentes boucles de régulation sensori-motrices au maintien de l'équilibre dans des conditions dynamiques.

A l'encontre de toutes les solutions actuelles qui obligent d'effectuer les analyses en conditions orthostatiques et dynamiques au moyen de plates-formes distinctes munies chacune de leurs propres capteurs de mesure et de leur propre unité d'acquisition et de traitement, invention a consisté à utiliser les potentialités offertes par les plates-formes statiques pour que ces dernières fassent office de moyens de mesure permettant d'évaluer les variations d'inclinaison qu'un individu impose à une plate-forme mobile, dans des conditions d'équilibration dynamique.

Pour un surcoût par rapport à une plate-forme statique correspondant au coût d'une simple plate-forme mobile, le dispositif d'analyse selon l'invention permet donc d'analyser la posture d'un individu en conditions orthostatiques en utilisant la seule plate-forme statique, et en conditions dynamiques en disposant la plate-forme mobile sur ladite plate-forme statique.

De plus, la plate-forme mobile étant positionnée sur une plate-forme statique, les capteurs de cette dernière permettent d'enregistrer non seulement les variations de l'inclinaison de ladite plate-forme mobile mais également les transferts d'appui selon un axe parallèle à l'axe du pivot. Ainsi de nombreux paramètres peuvent être exploités pour rendre compte des performances d'équilibration d'un sujet, tels que :
- la longueur du parcours d'équilibration exprimée en mm, degrés ou radians,
- l'amplitude maximale des oscillations exprimée en mm, degrés ou radians,
- la position moyenne de la plate-forme mobile autour de laquelle le sujet s'est équilibré, exprimée en mm, degrés ou radians,
- les composantes vectorielles selon deux axes x, y respectivement parallèle et orthogonal à l'axe du pivot, du parcours d'équilibration qui sont calculées et représentées en fonction du temps (stabilogramme en x, et stabiligramme en y).

Ces paramètres quantifient l'aptitude du sujet à s'équilibrer dans des conditions dynamiques.

D'autres paramètres permettent également de rendre compte des stratégies individuelles d'équilibration et d'apprécier la contribution relative des boucles de régulation sensori-motrices au maintien de l'équilibre du sujet.

Ces paramètres sont obtenus en effectuant une analyse fréquentielle de chaque stabilogramme par bandes de fréquence, c'est-à-dire en calculant l'énergie spectrale des oscillations avec indication de la répartition de cette énergie par bandes de fréquence et en pourcentage de l'énergie totale.

Concernant ces bandes de fréquence, et de façon avantageuse, l'unité d'acquisition et de traitement est programmée pour procéder à des analyses fréquentielles des oscillations et à leur objectivation dans trois bandes de fréquence, une bande de 0 à 0,5 Hz, une bande de 0,5 à 2 Hz et une bande de 2 Hz à 20 Hz.

La bande de 0 à 0,5 Hz correspond en effet aux boucles de régulation lentes à point de départ visuel et vestibulaire. La bande de 0,5 à 2 Hz correspond quant à elle aux boucles de régulation associées à l'intégration cérébelleuse. La bande de 2 Hz à 20 Hz correspond enfin aux boucles de régulation rapides associées aux réflexes myotatiques.

Selon un mode avantageux de réalisation, l'unité d'acquisition et de traitement est programmée pour acquérir les signaux délivrés par les capteurs de la plate-forme statique avec une fréquence supérieure ou égale à 40 Hz.. De telles fréquences d'acquisition permettent, en effet, d'obtenir des spectres idéaux dans une bande de fréquence de 0 à 11 Hz, cette dernière valeur constituant le tremblement physiologique le plus rapide d'un être humain.

Par ailleurs, le dispositif selon l'invention peut non seulement être utilisé avec un sujet en position debout, en vue de la mesure de son équilibre frontal et de son équilibre sagittal, mais également avec un sujet en position assise, moyennant de positionner et de sécuriser la plate-forme statique sur un support tel qu'un tabouret.

Dans ce cas, en outre, et de façon avantageuse, le dispositif comprend une assise de forme anatomique, ladite assise et la plate-forme mobile étant dotées de moyens de positionnement relatif de centrage et de maintien de l'assise sur la plate-forme mobile.

De plus, de façon avantageuse, les moyens de positionnement relatif de l'assise et de la plate-forme comprennent un orifice central ménagé dans le plateau de ladite plate-forme mobile, et une tige de centrage solidarisée sous ladite assise et de section adaptée pour se loger dans l'orifice dudit plateau.

Par ailleurs, selon un mode de réalisation avantageux, les moyens supports du plateau de la plate-forme mobile consistent en deux arceaux en forme de secteur circulaire disposés latéralement sous ledit plateau.

D'autres caractéristiques buts et avantages de l'invention ressortiront de la description détaillée qui suit en référence aux dessins annexés qui en représentent à titre d'exemple non limitatif un mode de réalisation préférentiel. Sur ces dessins :

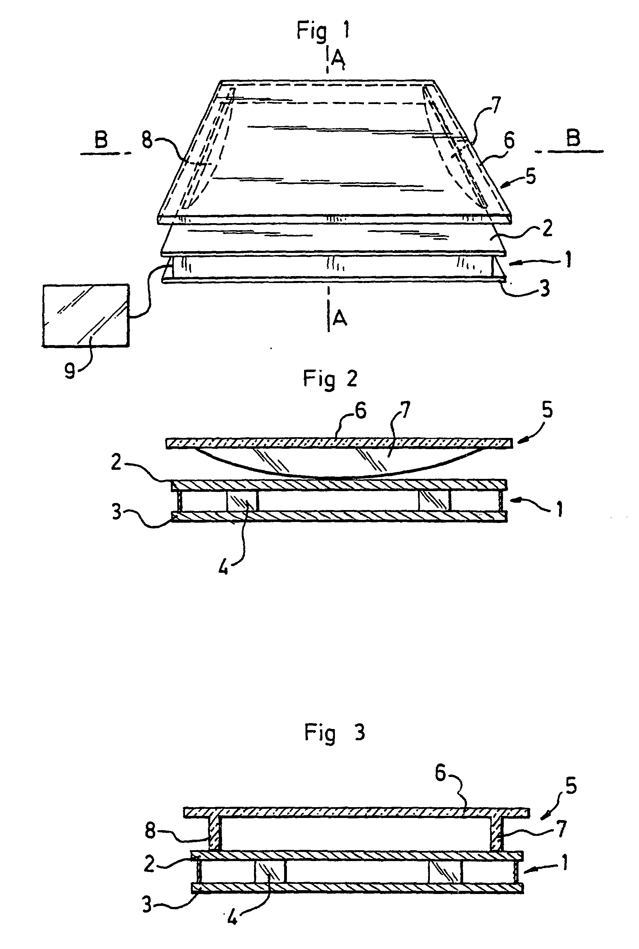

- la figure 1 est une vue en perspective d'un dispositif conforme à l'invention, d'analyse de la posture d'un individu en position debout,
- la figure 2 est une coupe transversale par un plan A de ce dispositif d'analyse,
- la figure 3 en est une coupe transversale par un plan B,
- la figure 4 est une vue en perspective d'un dispositif conforme à l'invention d'analyse de la posture d'un individu en position assise,
- et les figures 5a à 5c sont des diagrammes représentant certains des paramètres d'exploitation obtenus au moyen d'un dispositif d'analyse conforme à l'invention.

Le dispositif d'analyse de la posture d'un individu représenté aux figures est adapté pour permettre d'effectuer cette analyse dans des conditions orthostatiques et dans des conditions dynamiques, avec dans ce second cas, possibilité de mesurer l'équilibre frontal et l'équilibre sagittal de l'individu.

Ce dispositif comprend, en premier lieu, une plate-forme statique 1, composée de deux plateaux carrés parallèles 2, 3 entre lesquels sont disposés trois capteurs tels que 4, du type poutres à moment constant, uniformément répartis autour de l'axe desdits plateaux. Une telle plate-forme statique 1, utilisée seule, permet de façon classique d'analyser la posture d'un individu dans des conditions orthostatiques, et est reliée à cet effet à un micro-ordinateur 9 d'acquisition et de traitement des signaux délivrés par les capteurs.

Le dispositif comprend, en outre, une plate-forme mobile 5 destinée à être positionnée sur la plate-forme statique 1. Cette plate-forme mobile 5 est constituée d'un plateau carré 6 sous lequel sont fixés deux arceaux latéraux 7, 8 présentant un rayon de courbure de 55 cm et une flèche de 6 cm, qui confèrent à ladite plate-forme un seul degré d'inclination.

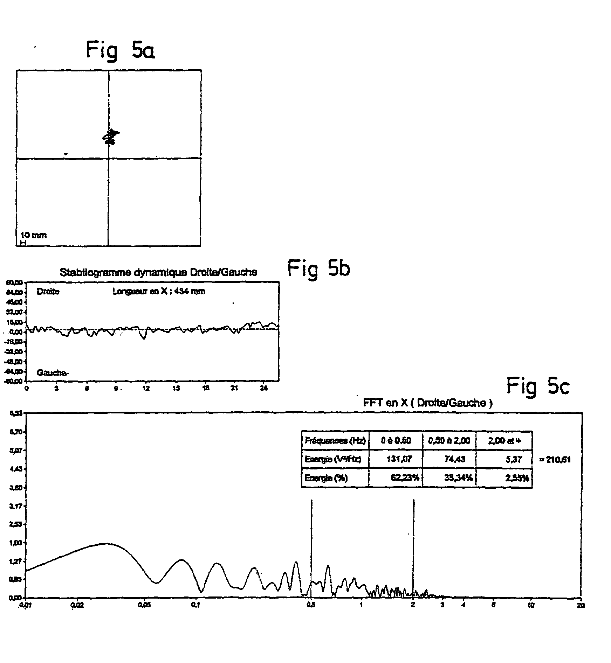

Lors d'une séance d'examen destinée à évaluer soit l'équilibre frontal, soit l'équilibre sagittal d'un individu, les signaux délivrés par les capteurs 4 sont acquis par le micro-ordinateur 9 avec une fréquence de 40 Hz, puis traités de façon à fournir les paramètres exploitables suivants :
- longueur du parcours d'équilibration avec tracé de ce dernier, tel que représenté à titre d'exemple à la figure 5a, ledit parcours et ledit tracé correspondant aux positions successives échantillonnées du centre de pression,
- l'amplitude maximale des oscillations,
- la position moyenne de la plate-forme mobile 1 autour de laquelle le sujet s'est équilibré,
- les composantes vectorielles en x et y du parcours d'équilibration qui sont calculées et tracées (stabilogramme en x et en y) en fonction du temps, tel que représenté à titre d'exemple à la figure 5b,
- les résultats de l'analyse spectrale (transformées rapides de FOURIER) de chaque stabilogramme qui permet de calculer l'énergie totale du spectre et l'énergie par bandes de fréquences. La figure 5c représente à titre d'exemple, un tracé de ces résultats qui met en lumière que trois bandes de fréquences sont étudiées, à savoir respectivement 0 - 0,5 Hz, 0,5 - 2 Hz, 2 - 20 Hz.

Le dispositif selon l'invention peut également être utilisé de façon à effectuer des mesures avec des patients en position assise, dans des conditions dynamiques, les pied ballants ou les pieds posés sur un marche-pied, dans le but d'une évaluation et d'une rééducation proprioceptive du rachis.

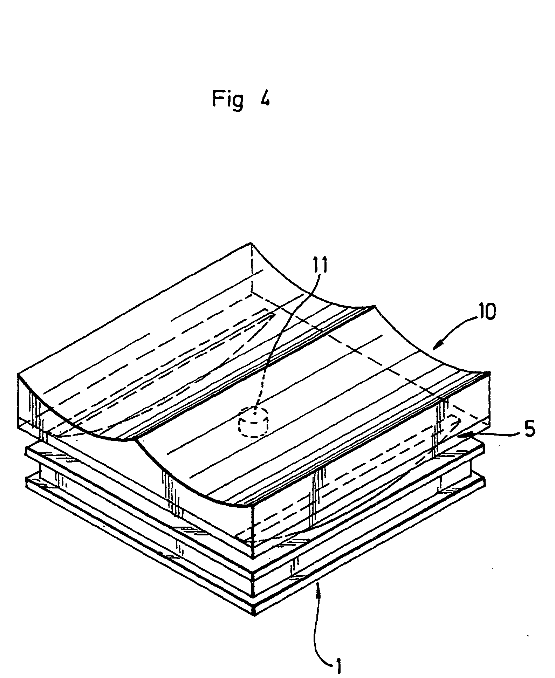

A cet effet, ce dispositif doit être disposé et sécurisé sur un tabouret. De plus, tel que représenté à la figure 4, à des fins ergonomiques, une assise 10 de forme anatomique est disposée sur le plateau 6 de la plate-forme mobile 5.

En outre, en vue d'assurer le positionnement de cette assise 10, cette dernière comporte une tige de centrage 11 solidarisée sous ladite assise, adaptée pour venir se loger dans un orifice axial ménagé dans le plateau 6.

## Revendications

1. Dispositif d'analyse de la posture d'un individu dans des conditions orthostatiques et/ou dynamiques, comprenant en combinaison :
- une plate-forme de forces statique (1) dotée de capteurs (4) aptes à délivrer des signaux représentatifs de la position et de la valeur absolue des forces exercées sur ladite plate-forme par un individu en situation d'équilibre orthostatique,
- une plate-forme mobile (5) disposée sur la plate-forme statique (1) et constituée d'un plateau (6) fixé sur des moyens supports (7, 8),
- une unité (9) d'acquisition et de traitement des signaux délivrés par les capteurs (4) de la plate-forme statique (1), programmée pour acquérir lesdits signaux avec une fréquence prédéterminée,
ledit dispositif étant **caractérisé en ce que** :
lesdits supports (7, 8) présentent une face inférieure d'appui en forme de secteur cylindrique dont la zone de contact avec la plate-forme statique (1) consiste en une des génératrices de ladite face inférieure d'appui formant le pivot de ladite plate-forme mobile, et
ladite unité d'acquisition et de traitement des signaux est également programmée pour procéder à une analyse des troubles de l'équilibre et de la posture de l'individu selon les plans d'équilibration frontal et sagittal, et à des analyses fréquentielles des oscillations engendrées de façon à définir la contribution des différentes boucles de régulation sensori-motrices au maintien de l'équilibre dans des conditions dynamiques.

2. Dispositif d'analyse selon la revendication 1 dans lequel les moyens supports du plateau (6) de la plate-forme mobile (5) consistent en deux arceaux (7, 8) en forme de secteur circulaire disposés latéralement sous ledit plateau.

3. Dispositif d'analyse selon l'une des revendications 1 ou 2, **caractérisé en ce que** l'unité d'acquisition et de traitement (9) est programmée pour acquérir les signaux délivrés par les capteurs (4) de la plate-forme statique (1) avec une fréquence supérieure ou égale à 40 Hz.

4. Dispositif d'analyse selon l'une des revendications 1 à 3 **caractérisé, en ce que** l'unité d'acquisition et de traitement (9) est programmée pour procéder à des analyses fréquentielles des oscillations objectives dans trois bandes de fréquences, une bande de 0 à 0,5 Hz, une bande de 0,5 à 2 Hz et une bande de 2 Hz à 20 Hz.

5. Dispositif d'analyse selon l'une des revendications 1 à 4 **caractérisé en ce qu'**il comprend une assise (10) de forme anatomique, ladite assise et la plate-forme mobile (5) étant dotés de moyens de positionnement relatif (11) de centrage et de maintien de l'assise (10) sur la plate-forme mobile (5).

6. Dispositif d'analyse selon la revendication 5, **caractérisé en ce que** les moyens de positionnement relatif de l'assise (10) et de la plate-forme (5) comprennent un orifice central ménagé dans le plateau (6) de ladite plate-forme mobile, et une tige de centrage (11) solidarisée sous ladite assise et de section adaptée pour se loger dans l'orifice dudit plateau.

## Patentansprüche

1. Vorrichtung zum Analysieren der Haltung einer Person unter orthostatischen und/oder dynamischen Bedingungen, wobei die Vorrichtung in Kombination Folgendes umfasst:
- eine statische Kraftmessplattform (1), die mit Sensoren (4) ausgestattet ist, die Signale erzeugen, die für die Position und den Absolutwert von Kräften repräsentativ sind, die von einer Person in einer Situation mit orthostatischem Gleichgewicht auf die genannte Plattform ausgeübt werden,
- eine bewegliche Plattform (5), die auf der statischen Plattform (1) angeordnet ist und von einer Platte (6) gebildet wird, die an Auflagemitteln (7, 8) befestigt ist,
- eine Einheit (9) zum Erfassen und Verarbeiten der Signale, die von den Sensoren (4) der statischen Plattform (1) erzeugt werden, die zum Erfassen der genannten Signale mit einer vorbestimmten Frequenz programmiert ist,
wobei die genannte Vorrichtung **dadurch gekennzeichnet ist, dass**:
die genannten Auflagen (7, 8) eine untere Abstützfläche in der Form eines Zylindersektors aufweisen, dessen Kontaktzone mit der statischen Plattform (1) aus einer der Erzeugenden der genannten unteren Abstützfläche besteht, die den Drehpunkt der genannten beweglichen Plattform bildet, und
die genannte Einheit zum Erfassen und Verarbeiten von Signalen auch programmiert ist, um eine Analyse von Gleichgewichts- und Haltungsstörungen der Person gemäß frontalen und sagittalen Gleichgewichtsebenen und Frequenzanalysen von Oszillationen durchzuführen, die erzeugt werden, um den Beitrag verschiedener sensomotorischer Regelschleifen zur Haltung des Gleichgewichts unter dynamischen Bedingungen zu definieren.

2. Analysevorrichtung nach Anspruch 1, bei der die Auflagemittel der Platte (6) der beweglichen Plattform (5) aus zwei kleinen Bogen (7, 8) in der Form eines Kreissektors bestehen, die lateral unter der genannten Platte angeordnet sind.

3. Analysevorrichtung nach Anspruch 1 oder 2, **dadurch gekennzeichnet, dass** die Erfassungs- und Verarbeitungseinheit (9) zum Erfassen der von den Sensoren (4) der statischen Plattform (1) erzeugten Signale mit einer Frequenz von gleich oder größer als 40 Hz programmiert ist.

4. Analysevorrichtung nach einem der Ansprüche 1 bis 3, **dadurch gekennzeichnet, dass** die Erfassungs- und Verarbeitungseinheit (9) so programmiert ist, dass sie objektive Oszillationsfrequenzanalysen in drei Frequenzbändern durchführt, einem Band von 0 bis 0,5 Hz, einem Band von 0,5 bis 2 Hz und einem Band von 2 bis 20 Hz.

5. Analysevorrichtung nach einem der Ansprüche 1 bis 4, **dadurch gekennzeichnet, dass** sie einen anatomisch geformten Sitz (10) umfasst, wobei der genannte Sitz und die bewegliche Plattform (5) mit einem Mittel (11) zur relativen Positionierung versehen sind, um den Sitz (10) auf der beweglichen Plattform (5) zu zentrieren und zu halten.

6. Analysevorrichtung nach Anspruch 5, **dadurch gekennzeichnet, dass** die Mittel zum relativen Positionieren des Sitzes (10) und der Plattform (5) eine mittlere Öffnung, die in der Platte (6) der genannten beweglichen Plattform vorgesehen ist, und einen Zentrierungsstab (11) aufweisen, der unter dem genannten Sitz befestigt ist und einen solchen Querschnitt hat, dass er in der Öffnung der genannten Platte aufgenommen werden kann.

## Claims

1. Device for analysis of the posture of a person in orthostatic and/or dynamic conditions, comprising in association:
. a static force platform (1) which is provided with sensors (4), which can emit signals which are representative of the position and absolute value of the forces exerted on the said platform by a person in a position of orthostatic balance;
. a mobile platform (5), which is placed on the static platform (1), and consists of a stand (6) which is secured to means for support (7, 8); and
. a unit (9) for acquisition and processing of the signals emitted by the sensors (4) of the static platform (1), programmed in order to acquire the said signals with a pre-determined frequency,
the said device being **characterised in that** :
the said supports (7,8) have a lower support surface in the form of a cylindrical section, the area of contact of which with the static platform (1) consists of one of the generatrices of the said lower support surface, which forms the pivot of the said mobile platform, and
the said unit for acquisition and processing of the signals is also programmed to proceed with an analysis of the problems with balance and posture of the person, according to frontal and sagittal balance planes, and with frequential analyses of the oscillations created, such as to define the contribution of the different sensory-motor regulation loops, towards maintaining balance in dynamic conditions.

2. Device for analysis according to claim 1, wherein the means for support of the stand (6) of the mobile platform (5) consist of two arches (7, 8) in the form of a circular section, disposed laterally beneath the said stand.

3. Device for analysis according to claim 1 or claim 2, **characterised in that** the unit for acquisition and processing (9) is programmed in order to acquire the signals emitted by the sensors (4) of the static platform (1), with a frequency of 40 Hz or more.

4. Device for analysis according to any one of claims 1 to 3, **characterised in that** the unit for acquisition and processing (9) is programmed to proceed with objective frequential analyses of the oscillations, in three frequency bands, i.e. a band of 0 to 0.5 Hz, a band of 0.5 Hz to 2 Hz, and a band of 2 Hz to 20 Hz.

5. Device for analysis according to any one of claims 1 to 4, **characterised in that** it comprises a seat (10) with an anatomical shape, the said seat and the mobile platform (5) being provided with means for relative positioning (11) of centring and maintenance of the seat (10) on the mobile platform (5).

6. Device for analysis according to claim 5, **characterised in that** the means for relative positioning of the seat (10) and of the platform (5) comprise a central aperture which is provided in the stand (6) of the said mobile platform, and a centring rod (11) which is secured beneath the said seat, and has a cross-section suitable for being accommodated in the aperture of the said stand.
